# EUROPEAN PATENT SPECIFICATION

(11) **EP 4 282 444 B1**
(45) Date of publication and mention of the grant of the patent: **15.10.2025**
(21) Application number: 23175577.8
(22) Date of filing: 26.05.2023
(51) Int. Cl.: A61L 27/18

(54) **FACILE FABRICATION OF A 3D-PRINTABLE (BIO)MATERIAL, USE AND METHOD OF MAKING THE SAME**
EINFACHE HERSTELLUNG EINES 3D-BEDRUCKBAREN (BIO)MATERIALS UND HERSTELLUNGSVERFAHREN DAFÜR
FABRICATION FACILE D'UNE UTILISATION DE MATÉRIAU (BIO) IMPRIMABLE EN 3D ET SON PROCÉDÉ DE FABRICATION

(30) Priority: 26.05.2022 US 202263365372 P
(43) Date of publication of application: 29.11.2023
(73) Proprietor: The Chinese University of Hong Kong, Shatin, New Territories (HK)
(72) Inventor: KER, Dai Fei Elmer, Hong Kong (HK); ZHANG, Xu, Hong Kong (HK); WANG, Dan, Hong Kong (HK)
(74) Representative: WSL Patentanwälte Partnerschaft mbB

(56) References cited:
- EP-A1- 0 802 208
- EP-A1- 2 687 188
- EGLIN DAVID ET AL: "Thiol-Containing Degradable Poly(thiourethane-urethane)s for Tissue Engineering", JOURNAL OF BIOMATERIALS SCIENCE. POLYMER EDITION., vol. 21, no. 4, 2 January 2010 (2010-01-02), NL, pages 477 - 491, XP093068534, ISSN: 0920-5063, DOI: 10.1163/156856209X424404

## Description

### CROSS-REFERENCE TO RELATED APPLICATION

This application claims the benefit of U.S. Provisional Patent Application Serial No. 63/365,372, filed May 26, 2022.

### BACKGROUND OF THE INVENTION

Injuries resulting in bone-tendon/bone-ligament or tendon/ligament ruptures are common during sports or other activities, and technical advancement is needed to improve repair results. For example, rotator cuff injuries are typically repaired by affixing suture anchors to humeral bone followed by suture-mediated repair in an effort to restore the contiguity of the bone-tendon unit. Unfortunately, repaired rotator cuff tissues experience retear rates ranging from 22% to 91%, and massive tears may be challenging to repair. Available natural and synthetic grafts exist but long-term clinical outcomes have produced mixed results. Repair and replacement materials that better mimic native tendon biomechanical properties, possess attributes that enhance surgical repair stability, and possess the capability to deliver pro-regenerative cues should improve outcomes.

Eglin, Griffon and Alini, 2012, Journal of Biomaterials Science, Polymer Edition, Thiol-Containing degradable poly(thiourethane-urethanes)s for tissue engineering, discloses the preparation of linear polymers, namely poly(thiourethane-urethane)s, from 1,6-hexamethylene diisocyanate, poly(ε-caprolacton)diols (PCLs) and bis(2-mercaptoethyl)ether with the overall aim to apply them in biodegradable and biocompatible materials for cartilage and bone tissue engineering.

In order to restore viable biomechanical function to injured rotator cuff tissues, grafts must, in addition to being biocompatible, also exhibit the following qualities: (i) approximate the mechanical properties of bone-tendon or tendon tissues over long durations to withstand physiological loads, (ii) exhibit high suture retention strength to support surgical repair, and (iii) possess the capability to deliver pro-regenerative cues to enhance tissue healing. Available grafts that are marketed as tendon substitutes include: anisotropically aligned collagen biotextiles; layered poly(L-lactic acid) scaffolds; electrospun nanofibers with crimped morphology; collagen scaffolds crosslinked via hypoxia and lysyl oxidase; decellularized tissue obtained from human or animal cadaveric sources and subjected to chemical crosslinking, porous polyurethane urea, woven poly(L-lactic acid) scaffolds; and tyramine substituted-hyaluronan enriched fascia extracellular matrix.. Available grafts possess tensile strengths (11.9-32.7 MPa) that span or exceed that of human tendon tissue, however, the tensile moduli (14 - 71 MPa) are typically 3 to 40 times lower than native supraspinatus tendon (200 - 600 MPa), which is the most frequently injured tendon in rotator cuff injuries and may affect efficiency of joint movement. It is vital to note that biomaterials may lose their mechanical properties as they degrade so biomaterials that do exhibit slow or no degradation are required to sustain mechanical attributes long-term. Also, few clinical grafts possess high suture retention, which is vital for ensuring surgical repair integrity. This is important as surgical sutures frequently tear through remaining tendon tissue and grafts may be used to physically reinforce the tendon. In addition, few grafts possess the capability to deliver pro-regenerative cues in terms of topography and growth factors. The former is vital for controlling cell alignment and ECM production, which in turn, affects tissue strength, while the latter is crucial for enhancing tissue regeneration. Furthermore, clinically available grafts are not mechanically tunable (to a particular tendon), are not produced on-site (at the hospital), and are not formed to the specific geometries of a patient's anatomy. Thus, tendon substitutes must possess necessary characteristics including biocompatibility, tendon-like mechanical properties, good suture-retention, and capability to deliver pro-regenerative cues that restore joint function and promote tissue healing, allowing a patient to resume daily activities.

To address this, a slow-degrading, photo-tunable polymer with robust tendon-like mechanical properties, high suture retention, and capability to deliver pro-regenerative cues was prepared by chemical-crosslinking, photo-crosslinking, and heat-curing. This was observed for a highly crosslinked, photo-tunable polythiourethane networks (PHT polymers) prepared from pentaerythritol tetra(3-mercaptopropionate) (P), hexamethylene diisocyanate (H) and trimethylolpropane triacrylate (T). PHT polymer is biocompatible and showed minimal cytotoxicity *in vitro* and no overt inflammatory response after implantation in mice for 3 weeks and in rabbits for 8 weeks. This polymer exhibits tendon-like strength and tensile modulus, the ability to sustain more than 10,000 cycles of physiologic loading without failure, and little-to-no degradation at 8 weeks post-implantation to retain mechanical strength. Also, PHT polymer exhibits exceptional suture retention properties, and showed reduced suture migration relative to GraftJacket^{™}, a commercially available acellular dermal matrix. In addition, PHT polymer is 3D-printable and can deliver pro-regenerative cues via topographic surface patterning and via combination with growth factors such as tenogenic fibroblastic growth factor-2 (FGF-2), transforming growth factor-β3, and insulin-like growth factor-1 (IGF-1).

3D-printing holds great potential for alleviating healthcare burdens through point-of-care manufacturing of personalized implants, therefore, a mechanically robust and biocompatible resins that can be easily manufactured at hospital locations without specialized apparatus is desirable.

### BRIEF SUMMARY OF THE INVENTION

An embodiment is directed to a biomaterial resin for musculoskeletal, bone and/or teeth repair that is a composition of a thiourethane oligomer and at least one (meth)acrylate monomer with a photoinitiator, wherein the thiourethane oligomer is the addition product of at least one thiol monomer and at least one isocyanate monomer. The thiol monomer is 2,5-dimercaptomethyl-1,4-dithiane, 2,3-dimercapto-1-propanol, 2-mercapto-ethylsulfide, 1,2,3-trimercaptopropane, ethylene glycol bis(thioglycolate), ethylene glycol *bis*(3-mercaptopropionate), pentaerythritol tetra(3-mercaptopropionate), trimethylolpropane *tris*(3-mercaptopropionate), pentaerythritol tetra(2-mercaptoacetate), trimethylolpropane *tris*(2-mercaptoacetate), 1,6-hexanedithiol, 1,2-benzenedithiol, 1,3-benzenedithiol, isophorone diurethane thiol, or any combinations thereof. The isocyanate monomer is isophorone diisocyanate, methylene dicyclohexyl diisocyanate, 2,4-diisocyanatotoluene, 4,4'-methylene bis-(cyclohexylisocyanate), hexamethylene diisocyanate, biuret of hexamethylene diisocyanate, hexamethylene diisocyanate isocyanurate trimer, hexamethylene diisocyanate uretdione, poly(hexamethylene diisocyanate), isophorone diisocyanate trimer, 1,3cyclohexane bis(methylisocyanate), and 2,2,4,-trimethyl hexamethylene diisocyanate, or any combination thereof. The at least one (meth)acrylate monomer is methyl acrylate, ethyl acrylate, methyl methacrylate, acrylic anhydride, acrylamide, methacrylamide, acrylic acid, and methacrylic acid, methacrylic anhydride, ethylene glycoldi(meth)acrylate, tetraethyleneglycol-di(meth)acrylate, poly(ethylene glycol)dimethacrylates, the condensation product of bisphenol A and glycidyl methacrylate, 2,2'-bis[4-(3-methacryloxy-2-hydroxypropoxy)-phenyl]propane, hexanediol di(meth)acrylate, tripropylene glycol di(meth)acrylate, butanediol di(meth)acrylate, neopentyl glycol di(meth)acrylate, diethylene glycol di(meth)acrylate, triethylene glycol di(meth)acrylate, dipropylene glycol di(meth)acrylate, allyl(meth)acrylate trimethylolpropane triacrylate, tricyclodecane dimethanol diacrylate, or any combinations thereof. In an exemplary embodiment of the biomaterial resin, the thiol monomer is pentaerythritol tetra(3-mercaptopropionate), the isocyanate monomer is hexamethylene diisocyanate, and the (meth)acrylate monomer is trimethylolpropane triacrylate. The thiol monomer and isocyanate monomer are combined in ratio of thiol to isocyanate functionality being equal to or greater than three to form a prepolymer solution. The thiourethane oligomer can include a catalyst, such as dibutyl tin laurate, triethanol amine, *N,N,N',N'*-*tetrakis*(2-hydroxypropyl)ethylenediamine, 1-[*N,N-bis*(2-hydroxyethyl)amino]-2-propanol, 4-[*N,N*-*bis*(2-hydroxyethyl)amino]benzaldehyde, and/or tri-*iso*propanolamine. The biomaterial resin includes a photocatalyst, such as, but not limited to 1,2-dicyclohexyl-4,4,5,5-tetramethylbiguanidium n-butyltriphenylborate (WPBG300) or benzophenone. The photocatalyst can be used with a photosensitizer, such as, but not limited to 2 -isopropylthioxanthone (IPTX). A solvent, such as acetone and/or dimethylsulfoxide can be used.

Another embodiment is directed to a method of making the biomaterial resin, above. The thiourethane oligomer is formed by the addition reaction within a mixture of the at least one thiol monomer and at least one isocyanate monomer and the thiourethane oligomer is combined with at least one (meth)acrylate monomer and at least one photoinitiator, and optionally at least one photosensitizer to form the biomaterial resin. The thiourethane oligomer is formed with a ratio of thiol units to isocyanate units of three or more. And oligomerizing can be carried in the presence of a catalyst.

Subsequently, according to an embodiment, a biomaterial network is formed from the biomaterial resin above. The biomaterial network is generated by placing the biomaterial resin in a receiving stage or within a delivering portion of a 3D-printer where the biomaterial resin is irradiated with UV or visible radiation to generate a polymerized portion and an unpolymerized portion. The unpolymerized portion can be separated from the polymerized portion to isolate the biomaterial network. The biomaterial network can be heated to increase the crosslinking density. An irradiating of the biomaterial resin or biomaterial network can be performed through a mask to form a gradient of crosslinking in the biomaterial network.

In another embodiment the biomaterial network formed by the method above is useful as a biomaterial device for repair or replacement of tendons, ligaments, bone, or teeth. The tendon substitute can have a tensile strength of about 3 MPa to about 28 MPa and a tensile modulus of about 21 MPa to about 844 MPa.

### BRIEF DESCRIPTION OF THE DRAWINGS

Fig. 1A shows a chart of tensile strength for PHT networks with thermal treatment for 2, 4, and 8 hours in comparison to the range displayed by a supraspinatus tendon (SST), according to embodiments.
Fig. 1B shows a chart of tensile modulus for PHT networks with thermal treatment for 2, 4, and 8 hours in comparison to the range displayed by a supraspinatus tendon (SST), according to embodiments.
Fig. 1C shows a chart of percentage of strain at failure for PHT networks with thermal treatment for 2, 4, and 8 hours, according to embodiments.
Fig. 2A shows a plot of strain vs cycling for PHT networks with thermal treatment for 2, 4, and 8 hours, according to embodiments.
Fig. 2B shows plots of dynamic, storage, and loss moduli vs cycling for PHT networks with thermal treatment 4 hours, according to an embodiment.
Fig. 2C shows a chart of cyclic creep for PHT networks with thermal treatment for 2, 4, and 8 hours, according to embodiments.
Fig. 3A shows a plot of the static creep for PHT networks with thermal treatment for 2, 4, and 8 hours relative to that of a clinically available tendon replacement (ADM), according to embodiments.
Fig. 3B shows a chart of the creep rate for PHT networks with thermal treatment for 2, 4, and 8 hours relative to that of a clinically available tendon replacement (ADM), according to embodiments.
Fig. 3C shows a chart of the maximum strain change (%) in static creep for PHT networks with thermal treatment for 2, 4, and 8 hours relative to that of a clinically available tendon replacement (ADM), according to embodiments.
Fig. 3D shows a chart of the creep recovery (%) for PHT networks with thermal treatment for 2, 4, and 8 hours relative to that of a clinically available tendon replacement (ADM), according to embodiments.
Fig. 4 shows a chart of suture retention for a 4h-PHT network relative to that of a clinically available tendon replacement (ADM), according to an embodiment.
Fig. 5 shows picrosirius red staining and quantification of collagen expression of differentiated and control human mesenchymal stem cells cultured on the surface of the PHT networks with thermal treatment for 2, 4, and 8 hours, according to embodiments.

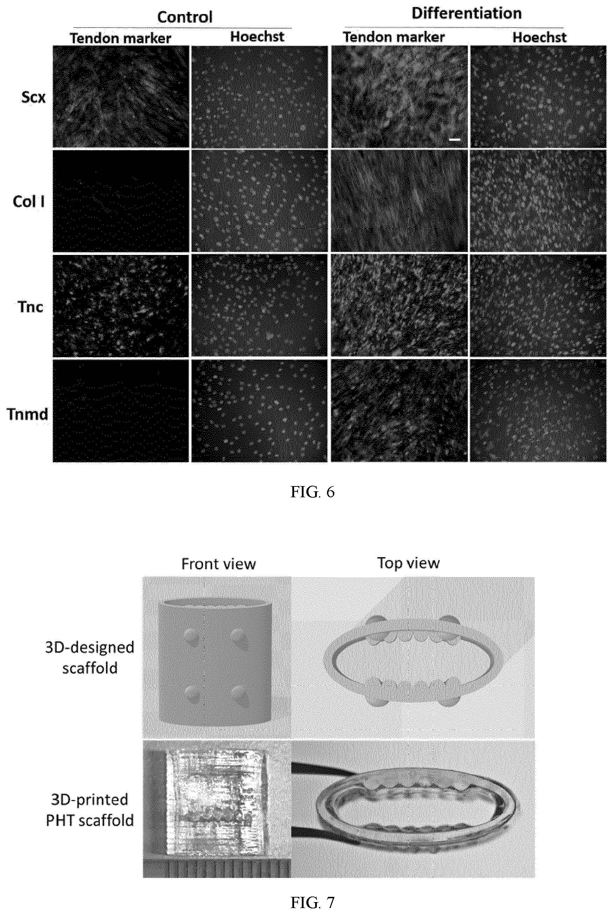

Fig. 6 shows immunofluorescence staining of tendon-specific markers Scleraxis (Scx), Collagen Type I (Col I), Tenascin C (Tnc) and Tenomodulin (Tnmd) on differentiated and control human mesenchymal stem cells cultured on the surface of the PHT networks with thermal treatment for 4 hours, according to an embodiment.
Fig. 7 shows the 3D-printed tunnel-shaped PHT network with an anisotropic topographical pattern inside.

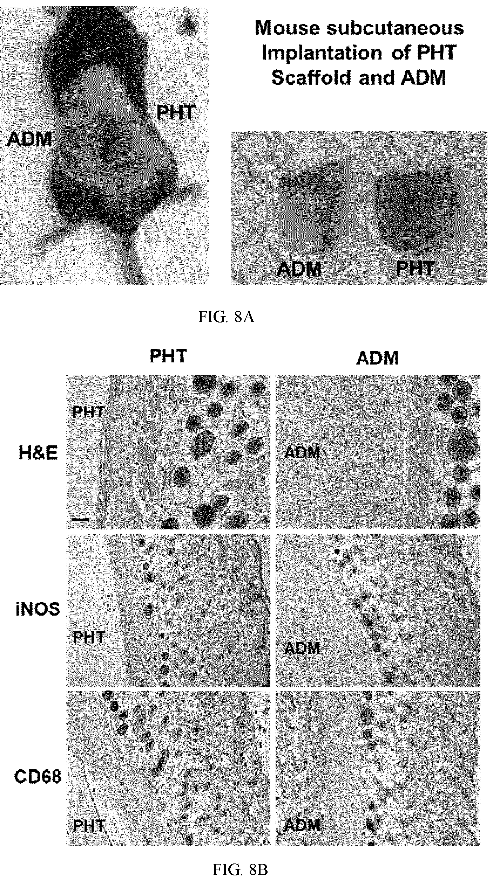

Fig. 8A shows mouse subcutaneous implantation of PHT network and a clinically available tendon replacement (ADM), according to embodiments.
Fig. 8B shows the hematoxylin and eosin (H&E) staining and immunohistochemistry of iNOS and CD68 on the skin sections with implanted PHT network and a clinically available tendon replacement (ADM).

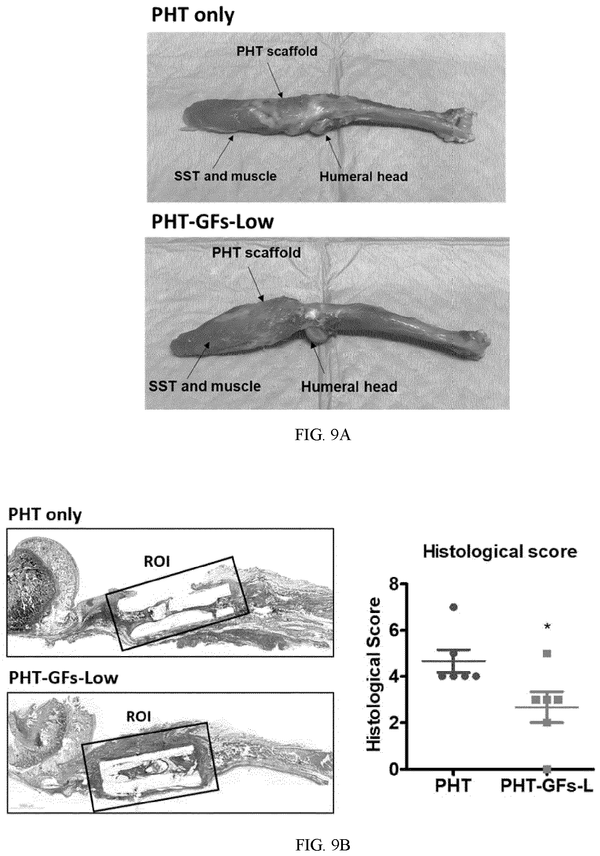

Fig. 9A shows gross tissue morphology of rabbit supraspinatus tendon implanted with PHT scaffold with thermal treatment for 4 hours, with or without low dosage of growth factors (GFs-Low) collected 8 weeks after surgery, according to an embodiment.
Fig. 9B shows images of hematoxylin and eosin staining of longitudinal sections of rabbit supraspinatus tendon with PHT only (with thermal treatment for 4 hours,) or PHT-GFs-Low (with thermal treatment for 4 hours), according to an embodiment, and the Histological score calculated according to defined criteria in the region of interest (ROI).

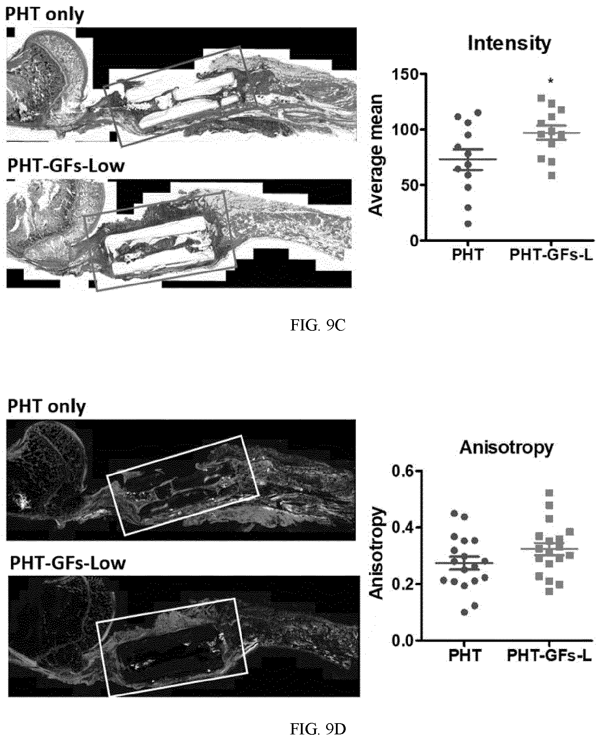

Fig. 9C shows images of picrosirius red staining of longitudinal sections of rabbit supraspinatus tendon with PHT only (with thermal treatment for 4 hours) or PHT-GFs-Low (with thermal treatment for 4 hours), according to an embodiment, and a chart of the Collagen expression in the ROI calculated as the average intensity of the red color of the fibers around the PHT scaffold.
Fig. 9D shows images of picrosirius red staining of longitudinal sections of rabbit supraspinatus tendon with PHT only (with thermal treatment for 4 hours) or PHT-GFs-Low (with thermal treatment for 4 hours), according to an embodiment, under polarized light and a chart of Collagen alignment calculated with anisotropy of the fibers around the PHT scaffold.

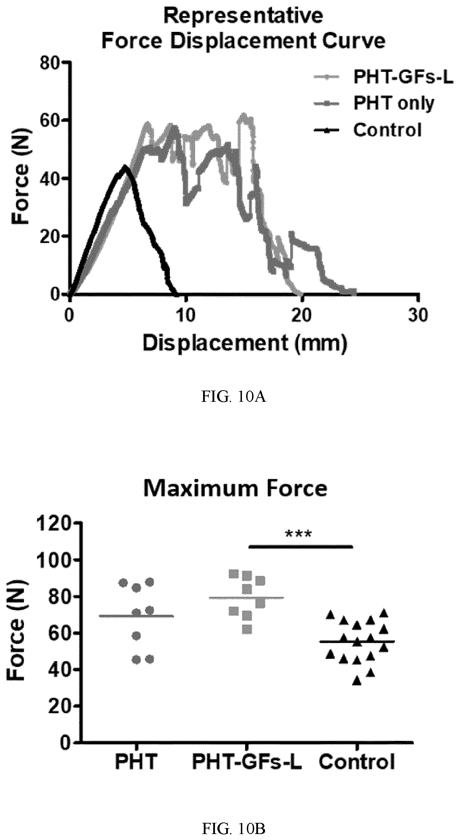

Fig. 10A shows representative force displacement profile of rabbit supraspinatus tendon with PHT only (with thermal treatment for 4 hours), PHT-GFs-Low (with thermal treatment for 4 hours), according to an embodiment, and contralateral control during mechanical tensile test.
Fig. 10B shows maximum force of rabbit supraspinatus tendon with PHT only (with thermal treatment for 4 hours), PHT-GFs-Low (with thermal treatment for 4 hours), according to an embodiment, and contralateral control in mechanical tensile test (load to failure).

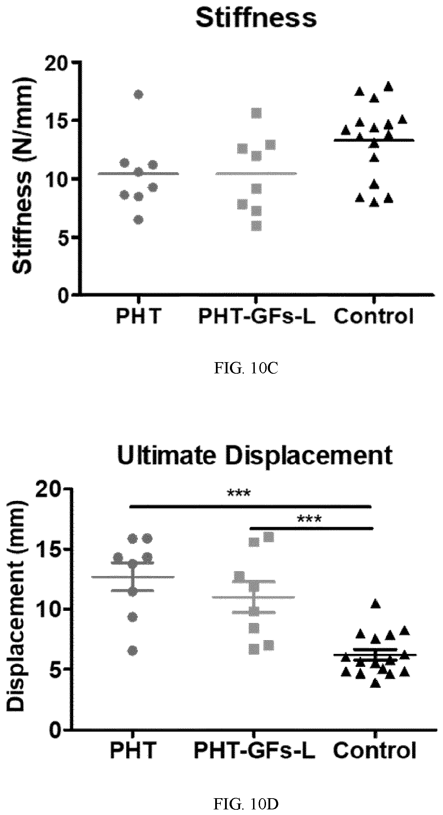

Fig. 10C shows stiffness of rabbit supraspinatus tendon with PHT only (with thermal treatment for 4 hours), PHT-GFs-Low (with thermal treatment for 4 hours), according to an embodiment, and contralateral control in mechanical tensile test (load to failure).
Fig. 10D shows ultimate displacement of rabbit supraspinatus tendon with PHT only (with thermal treatment for 4 hours), PHT-GFs-Low (with thermal treatment for 4 hours), according to an embodiment, and contralateral control in mechanical tensile test (load to failure).

### DETAILED DISCLOSURE OF THE INVENTION

In embodiments, a 3D-printable biomaterial resin for a mechanically robust tendon repair. This 3D-printed biomaterial allows point-of-care manufacturing of a biomaterial network for personalized tendon implants. The biomaterial resin has a formulation that is easily 3D-printed by a medical technician and yields a durable repair of musculoskeletal injuries, such as, but not limited to, rotator cuff tears, yet is applicable to other biomedical and non-biomedical devices. In embodiments of the invention the biomaterial resin is readily formulated with no specialized equipment or conditions. In embodiments, the biomaterial resin forms biomaterial network that exhibits mechanical properties similar to native tendons and ligaments that are highly biocompatible and can be 3D-printed to specific geometries that match specific patient anatomy. The biomaterial networks can possess stress reducing features via modulation of stiffness gradients or 3D-printed lattice structure.

The biomaterial resin has a composition that is ultimately formed from at least one multifunctional thiol monomer, at least one multifunctional isocyanate, and at least one (meth)acrylate monomer. The formulation is not typical of traditional polyurethanes where the formulation diminishes any risks of isocyanate-related toxicity to those formulating, transforming, or receiving a biomedical device from the biomaterial resin.

The biomaterial resin includes isocyanate monomers that contain at least two isocyanate functionalities. The isocyanate monomers are one or more of: isophorone diisocyanate; methylene dicyclohexyl diisocyanate; 2,4-diisocyanatotoluene; 4,4'-methylene-bis-(cyclohexylisocyanate); hexamethylene diisocyanate; biuret of hexamethylene diisocyanate; hexamethylene diisocyanate isocyanurate trimer; hexamethylene diisocyanate uretdione; poly(hexamethylene diisocyanate); isophorone diisocyanate trimer; 1,3-cyclohexane-*bis*-(methylisocyanate); and 2,2,4,-trimethylhexamethylene diisocyanate.

The biomaterial resin includes thiol monomers that contain at least two thiol functionalities. These thiol monomers are one or more of : 2,5-dimercaptomethyl-1,4-dithiane; 2,3-dimercapto-1-propanol; 2-mercapto-ethylsulfide; 1,2,3-trimercaptopropane; ethylene glycol *bis*(thioglycolate); ethylene glycol *bis*(3-mercaptopropionate); pentaerythritol *tetra*(3-mercaptopropionate); trimethylolpropane *tris*-3-mercaptopropionate); pentaerythritol *tetra*(2-mercaptoacetate); trimethylolpropane *tris*(2-mercaptoacetate); 1,6-hexanedithiol; 1,2-benzenedithiol; 1,3-benzenedithiol; and isophorone diurethane thiol.

The biomaterial resin includes (meth)acrylate monomers including one or more of: methyl acrylate; ethyl acrylate; methyl methacrylate; acrylic anhydride; acrylamide; methacrylamide; acrylic acid; and methacrylic acid; methacrylic anhydride; ethylene glycoldi(meth)acrylate; tetraethyleneglycol-di(meth)acrylate; poly(ethylene glycol)dimethacrylates; the condensation product of bisphenol A and glycidyl methacrylate; 2,2'-bis[4-(3-methacryloxy-2-hydroxypropoxy)-phenyl]propane; hexanediol di(meth)acrylate; tripropylene glycol di(meth)acrylate; butanediol di(meth)acrylate; neopentyl glycol di(meth)acrylate; diethylene glycol di(meth)acrylate; triethylene glycol di(meth)acrylate; dipropylene glycol di(meth)acrylate; allyl(meth)acrylate trimethylolpropane triacrylate; and tricyclodecane dimethanol diacrylate.

In embodiments, the isocyanate monomers and thiol monomers are combined such that the ratio of -SH to -NCO functionality is at least three. This results in a thiourethane oligomer that is effectively free of isocyanate units and containing at least two thiol units on each homologue of the oligomer. The oligomerization can include a catalyst, such as, but not limited to, butyl tin laurate, or a tertiary amine, such as, but not limited to: *N,N,N',N'*-*tetrakis*(2-hydroxypropyl)ethylenediamine; triethanol amine; triisopropanolamine; 1-[*N,N-bis*(2-hydroxyethyl)amino]-2-propanol, and 4-[N,N-bis(2-hydroxyethyl)amino]benzaldehyde. The oligomerization can be performed at ambient temperature and pressures, although heating can be performed as desired. The final thiourethane oligomer is retained at room temperature until subsequent inclusion into the biomaterial resin.

This thiourethane oligomer is combined with the (meth)acrylate monomers to form the biomaterial resin. The biomaterial resin can be combined at ambient temperature and pressure, although heating can be performed as desired. The mixture can but does not require a solvent. The mixture can include, during a single mixing or in a subsequent mixing to the oligomer-(meth)acrylate mixing, a photoinitiation component that includes a photoinitiator and, optionally, a photosensitizer, such a wide range of electromagnetic wavelengths can be used with the biomaterial resin during 3D printing and subsequent photolytic processes. The photoinitiator can be, but is not limited to, WPBG300, benzophenone, and an optional photosensitizer can be, but is not limited to IPTX. As desired or required, mixing can occur in the presence of a solvent, such as, but not limited to, acetone and/or dimethylsulfoxide (DMSO). The solvent can be included in the biomaterial resin or may be removed to yield the biomaterial resin in the form desired for subsequent processing into the biomaterial network. Additionally, if desired for a particular application, a pigment or dye can be included. As desired a filler can be combined with the biomaterial resin in a wide range of filler loadings. The filler can be, but is not necessarily, transparent to UV or visible light. The filler can be of any reasonable dimensions from nanometer to greater than micrometer dimensions. Fillers can include, but are not limited to, silica, glass, mineral, polymeric particles or fibers, graphite fibers, carbon nanotubes.

The biomaterial resin forms a biomaterial network when placed in a 3D printer. The biomaterial resin is then patterned by photolysis using the printer's lasers. The printed biomaterial resin can be subsequently heated as desired. The resulting poly((meth)acrylate thiourethane) forms the biomaterial network. The (meth)acrylate polymerizes with the thiourethane oligomer acting as a chain transfer reagent to connect (meth)acrylate and thiol chains within the network. This biomaterial network can be used as achieved after removal of non-polymerized resin, or the isolated biomaterial network can be additionally processed to form a biomaterial network that has a higher crosslinking density and can have a gradient of crosslinking densities to yield a biomaterial device with a gradient of mechanical properties along at least one dimension of the biomaterial device. The biomaterial device can be heated to increase the physical crosslinking density of the biomaterial. Heating can be performed in an oven or on a plate such that heat is maintained for a desired length of time to achieve desired tensile strengths and tensile moduli for a desired use of the device.

The gradient biomaterial device can be generated using a UV or visible light source in conjunction with a mask. The mask can be included in the 3D-printing process or subsequently with the biomaterial device. The mask can be used in a stationary or moving manner, such that the desired gradient can be achieved. The mask can be continuously transmissive, or maybe formed to have a desired gradient of transmissivity.

In an exemplary embodiment, a biomaterial resin was prepared from a thiourethane oligomer from pentaerythritol tetra(3-mercaptopropionate) and hexamethylene diisocyanate in a 1.5 to 1 molar ratio (3 -SH / -NCO) to form approximately a pentameric thiourethane oligomer. This thiourethane oligomer can be combined with the trimethylolpropane triacrylate and catalyst to form a biomaterial network upon irradiating in a 3D-printer. The triacrylate forms a network that includes the pentameric thiourethane oligomer by chain transfer during the radical chain-growth polymerization of the acrylate units. Polymerization proceeds until the network forms with little diffusion within the network. Heating of this biomaterial network increase diffusion and the crosslinking density of the network. This network can be fabricated as a biomaterial device that displays a broad range of tensile strengths of about 3 to 28 MPa, for example, 3.06 MPa when no heating is employed to about 27.53 MPa when the equivalent biomaterial network is heated for eight hours, and a broad range of tensile modulus of about 20 to about 844 MPa, for example, 20.54 MPa for the non-heated biomaterial network and 843.7 MPa for the equivalent biomaterial network is heated for eight hours.

The biomaterial device or gradient biomaterial is useful as a tendon substitute or other biomaterials for repair or replacement of tendons, ligaments, bone, or teeth as formulated by the photolytic or combined photolytic and thermal processes.

### METHODS AND MATERIALS

Pentaerythritol tetra(3-mercaptopropionate) (Sigma Aldrich), hexamethylene diisocyanate (1,6-diisocyanatohexane) ( TCI (Shanghai) Development Co., Ltd), trimethylolpropane triacrylate (stabilized with MEHQ) (Sigma), *N,N,N',N'-tetrakis*(2-hydroxy-propyl) ethylenediamine (quadrol or Q; Sigma Aldrich), 1,2-dicyclohexyl-4,4,5,5-tetramethylbiguanidium n-butyltriphenylborate (WPBG-300; FUJIFILM Wako Pure Chemical Co.), 2-isopropylthioxanthone (FUJIFILM Wako Pure Chemical Co.), and acetone (Honeywell International Inc) were used as received. A Kudo3D Titan 2 HR 3D printer or a 365 nm UV light source (365 nm UV LED lamp; 6000-8000MW/cm²; ShenZhen HowSuper Technology Co. Ltd) were employed.

A biomaterial resin (PHT) was prepared by combining the thiol and isocyanate to produce PH oligomer solution with excess thiol, where 50.03 mmol pentaerythritol tetra(3-mercaptopropionate) (P) was mixed with 10 µL *N,N,N',N'-tetrakis*(2-hydroxy-propyl) ethylenediamine (quadrol or Q) solution (33.3% v/v, in acetone) as catalyst in a 50 mL conical tube with vortexing for 30 seconds for mixing. Addition of 19.92 mmol hexamethylene diisocyanate (H) to the mixture and vortexing at 2500rpm at RT for 20 to 30 minutes to allow complete reaction of H and generate a PH oligomer where ratio of -SH to -NCO groups was 5:1. The PH oligomer was centrifuged at 2200 g for 3 minutes to remove bubbles and 80.18 mmol trimethylolpropane triacrylate (T) was added. To the PH oligomer T mixture was added 5 mL acetone and physically mixed with a spoon until a homogeneous solution was formed (SH:NCO:Acrylate = 5:1:6).

To this PHT resin was added 500 µL 1,2-dicyclohexyl-4,4,5,5-tetramethylbiguanidium n-butyltriphenylborate (WPBG-300) solution (10% w/v, in acetone) and 100 µL 2 - isopropylthioxanthone (IPTX) solution (10% w/v, in acetone) as photo initiator and sensitizer. The mixture was centrifuged at 2200 g for 0.5 minutes and transferred into a modified ASTM D638-10 Type V mold. A mold was placed on the platform of a Kudo3D printer Titan 2 HR and the PHT resin was photo-crosslinked for 150 seconds.

PHT networks were removed from the mold and additionally heat cured in a vacuum oven (Shel Lab SVAC1-2 Compact Vacuum Drying Oven, Albuquerque, New Mexico) at 150 °C for 2, 4, or 8 hours. Mechanical properties of PHT networks were determined by tensile, cyclic tensile, static creep and suture retention tests.

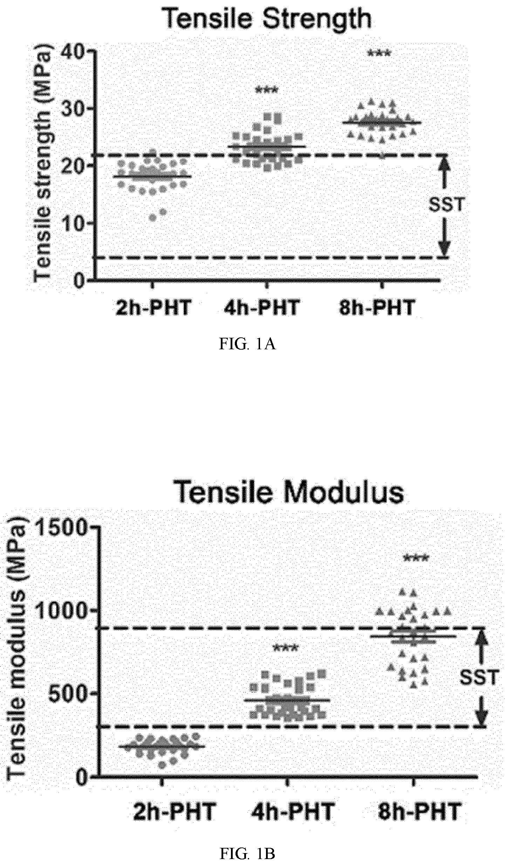

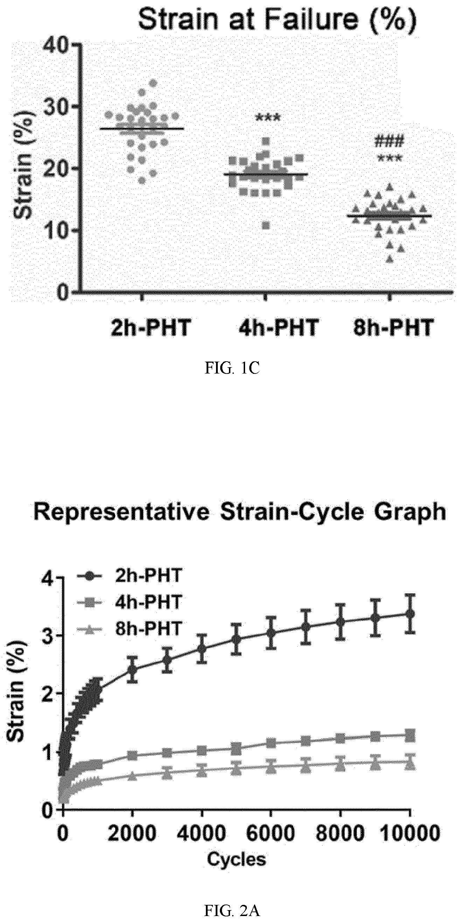

Tensile test (load to failure) indicated that the PHT network has a tensile strength range of 3.06 MPa with no heat-curing to about 27.53 MPa with 8hrs heat-curing (8h-PHT) and a tensile modulus range of 20.54 MPa with no heat-curing to about 843.7 MPa for 8h-PHT. The PHT network showed increased tensile strength and tensile moduli with increased heat-curing time and is capable to mimic physiological supraspinatus tendon-like strength and modulus, as indicated in Figs. 1A-1C.

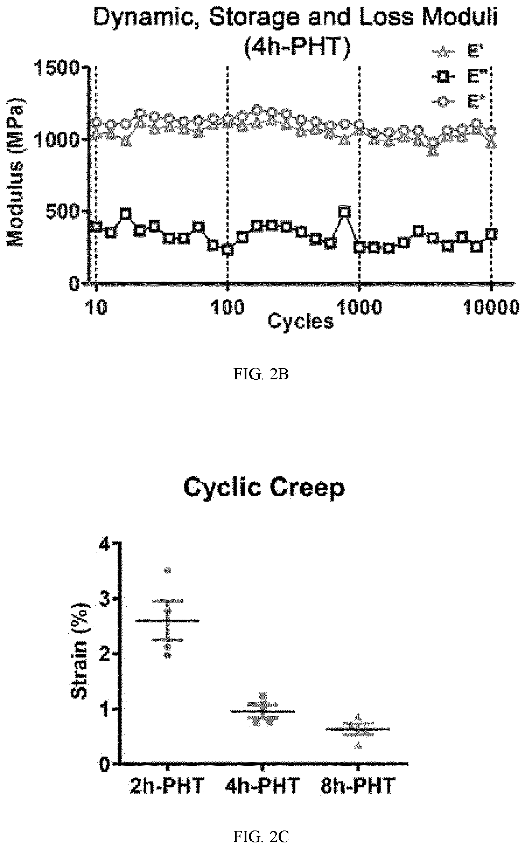

Cyclic tensile test were conducted on the PHT network, which exhibited: strain of 2.59 ± 0.35 % (2hrs heat-curing; 2h-PHT), 0.96 ± 0.12 % (4hrs heat-curing; 4h-PHT), and 0.64 ± 0.10 % 8h-PHT, as shown in Fig. 2C; dynamic moduli for 2h-PHT (705.7 ± 2.126 MPa), 4h-PHT (1113 ± 9.670 MPa), and 8h-PHT (1369 ± 8.276 MPa); storage moduli for 2h-PHT (672.4 ± 2.699 MPa), 4h-PHT (1052 ± 9.797 MPa) and 8h-PHT (1306± 9.113 MPa); and loss moduli for 2h-PHT (196.9 ± 7.260 MPa), 4h-PHT (335.8 ± 13.09 MPa), and 8h-PHT (363.2 ± 22.85 MPa), as shown in Fig. 2B, during 10,000 loading cycles from 0.2 to 3 MPa tensile stress at 1 Hz. The change of strain (%) cyclic creep decreased with increased heat-curing time of the PHT networks, as shown in Fig. 2C. These results indicate that the PHT networks have robust tensile properties that withstand at least 10000 cycles of physiologic loading.

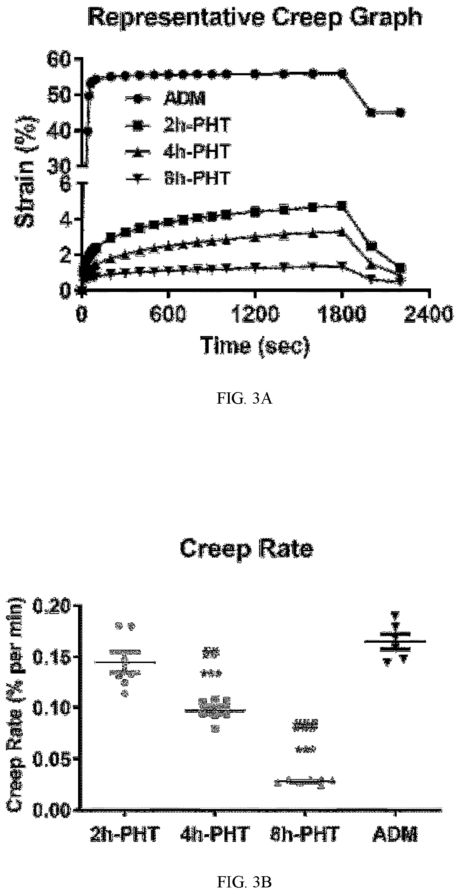

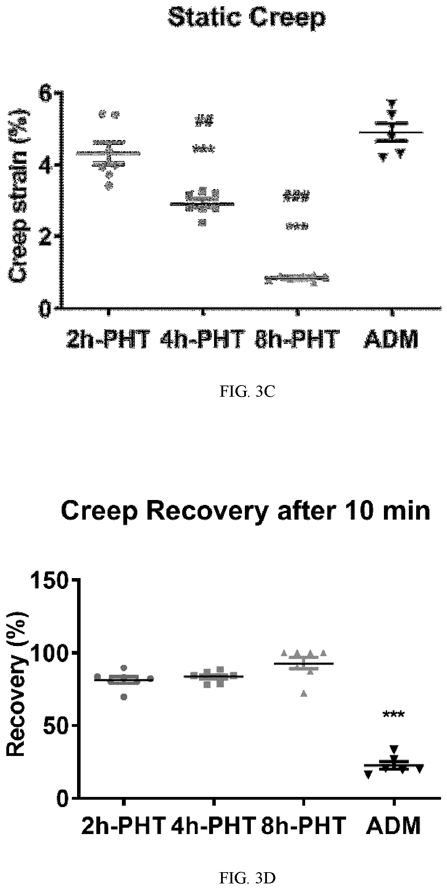

Static creep testing of PHT networks exhibited 4.323 ± 0.2993 % (2h-PHT), 2.918 ± 0.1167 % (4h-PHT) and 0.8478 ± 0.0263 % (8h-PHT) strain following a 30 min hold at 3 MPa tensile stress and recovered for 80.89 ± 2.300 %, 83.01 ± 1.450 % and 92.71 ± 4.039 % following a 10 min recovery period for 2h-, 4h- and 8h-PHT, respectively, as indicated in Fig. 3A, which was superior to that displayed by a clinically available ADM. The creep rate is shown in Fig. 3B relative to ADM for 2h-, 4h-, and 8h-PHT were 0.1441 ± 0.0099 %, 0.09726 ± 0.0038 %, and 0.02823 ± 0.00087 % per min, respectively. Furthermore, the 4h-PHT and 8h-PHT showed less strain (%) change for the static creep during the 30min hold at 3 MPa, as shown in Fig. 3C, and greater percentage of creep recovery after 10 min, as shown in Fig. 3D compared to the clinically available ADM demonstrating its robust fatigue and recovery properties that can be achieved with PHT networks.

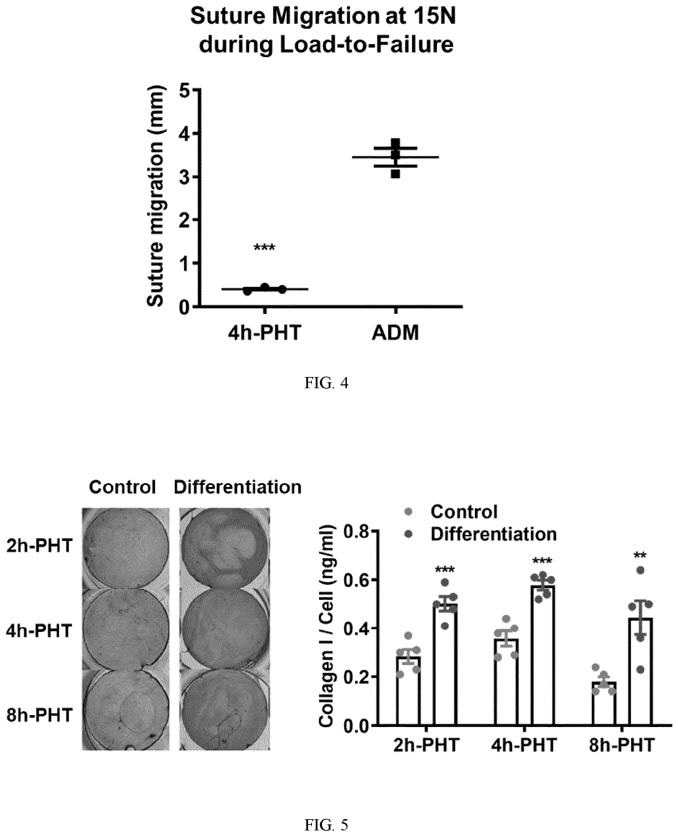

A suture retention test was performed on a PHT network after 4h heat-curing, exhibiting an 8.56-fold smaller suture migration during a pull-to-failure (at 15 N) relative to the clinically available ADM, as shown in Fig. 4. The suture migration for the 4h-PHT, being less than 0.5 mm is excellent relative to state-of-the-art tendon substitutes.

## Claims

1. A biomaterial resin for musculoskeletal, bone and/or teeth repair comprising a thiourethane oligomer, at least one (meth)acrylate monomer and at least one photoinitiator, wherein the thiourethane oligomer comprises an addition product of at least one thiol monomer, and at least one isocyanate monomer, wherein the at least one thiol monomer comprises 2,5-dimercaptomethyl-1,4-dithiane; 2,3-dimercapto-1-propanol; 2-mercapto-ethylsulfide; 1,2,3-trimercaptopropane; ethylene glycol bis(thioglycolate); ethylene glycol *bis*(3-mercaptopropionate); pentaerythritol tetra(3-mercaptopropionate); trimethylolpropane *tris*(3-mercaptopropionate); pentaerythritol tetra(2-mercaptoacetate); trimethylolpropane *tris*(2-mercaptoacetate); 1,6-hexanedithiol; 1,2-benzenedithiol; 1,3-benzenedithiol; isophorone diurethane thiol; or any combination thereof, wherein the at least one isocyanate monomer comprises isophorone diisocyanate; methylene dicyclohexyl diisocyanate; 2,4-diisocyanatotoluene; 4,4'-methylene bis-(cyclohexylisocyanate); hexamethylene diisocyanate; biuret of hexamethylene diisocyanate; hexamethylene diisocyanate isocyanurate trimer; hexamethylene diisocyanate uretdione; poly(hexamethylene diisocyanate); isophorone diisocyanate trimer; 1,3cyclohexane bis(methylisocyanate); 2,2,4,-trimethyl hexamethylene diisocyanate; or any combination thereof, and wherein the at least one (meth)acrylate monomer comprises methyl acrylate; ethyl acrylate; methyl methacrylate; acrylic anhydride; acrylamide; methacrylamide; acrylic acid; methacrylic acid; methacrylic anhydride; ethylene glycoldi(meth)acrylate; tetraethyleneglycol-di(meth)acrylate; poly(ethylene glycol)dimethacrylate; a condensation product of bisphenol A and glycidyl methacrylate; 2,2'-bis[4-(3-methacryloxy-2-hydroxypropoxy)-phenyl]propane; hexanediol di(meth)acrylate; tripropylene glycol di(meth)acrylate; butanediol di(meth)acrylate; neopentyl glycol di(meth)acrylate; diethylene glycol di(meth)acrylate; triethylene glycol di(meth)acrylate; dipropylene glycol di(meth)acrylate; allyl(meth)acrylate trimethylolpropane triacrylate; tricyclodecane dimethanol diacrylate; or any combination thereof,.

2. The biomaterial resin according to claim 1, wherein the thiol monomer is pentaerythritol tetra(3-mercaptopropionate), the isocyanate monomer is hexamethylene diisocyanate, and the (meth)acrylate monomer is trimethylolpropane triacrylate.

3. The biomaterial resin according to claim 1, wherein the thiol monomer and isocyanate monomer are combined in ratio of thiol to isocyanate equal to or greater than three.

4. The biomaterial resin according to claim 1, wherein the thiourethane oligomer further comprises a catalyst, preferably wherein the catalyst comprises dibutyl tin laurate; *N,N,N',N'-tetrakis*(2-hydroxypropyl)ethylenediamine; 1-[*N,N-bis*(2-hydroxyethyl)amino]-2-propanol; 4-[*N,N-bis*(2-hydroxyethyl)amino]benzaldehyde; triethanol amine; tri-isopropanolamine; or any combination thereof.

5. The biomaterial resin according to claim 1, further comprising a photocatalyst such as 1,2-dicyclohexyl-4,4,5,5-tetramethylbiguanidium n-butyltriphenylborate; benzophenone; or any combination thereof.

6. The biomaterial resin according to claim 1, further comprising a photosensitizer and/or a solvent, preferably wherein the photosensitizer comprises 2 -isopropylthioxanthone and preferably,
wherein the solvent is acetone or dimethylsulfoxide.

7. A method of making a biomaterial resin according to claim 1, comprising:
oligomerizing a mixture comprising at least one thiol monomer and at least one isocyanate monomer to form a thiourethane oligomer; and
combining the thiourethane oligomer with at least one (meth)acrylate monomer and at least one photoinitiator, and optionally at least one photosensitizer to form the biomaterial resin, optionally wherein the oligomerizing is in the presence of a catalyst.

8. The method according to claim 7, wherein the mixture comprises a ratio of thiol units to isocyanate units of three or more.

9. A method of forming a biomaterial network, comprising:
providing a biomaterial resin according to claim 1;
placing the biomaterial resin in a resin receiving or delivering stage of a 3D-printer;
irradiating the biomaterial resin with UV or visible radiation to generate a polymerized portion and an unpolymerized portion;
removing the unpolymerized portion from the polymerized portion to isolate the biomaterial network, and
optionally heating the biomaterial network.

10. The method according to claim 9, further comprising irradiating the biomaterial resin with UV or visible light of the biomaterial network thorough a mask.

11. The method according to claim 10, further comprising: changing the UV or visible light radiation dosage relative to the biomaterial resin and/or the biomaterial network: or the position of the mask relative to the biomaterial resin and/or the biomaterial network, whereby the biomaterial network has a gradient of crosslink density.

12. A biomaterial device for repair or replacement of tendons, ligaments, bone, or teeth, preferably, wherein the biomaterial device is a tendon substitute, comprising a biomaterial network formed by the method according to claim 9.

13. The biomaterial device according to claim 12, wherein the biomaterial network has a tensile strength of about 3 MPa to about 28 MPa and a tensile modulus of about 21 MPa to about 844 MPa.

## Patentansprüche

1. Biomaterialharz zur Reparatur von muskuloskelettalem Gewebe, Knochen und/oder Zähnen, umfassend ein Thiourethan-Oligomer, mindestens ein (Meth)acrylat-Monomer und mindestens einen Photoinitiator, wobei das Thiourethan-Oligomer ein Additionsprodukt aus mindestens einem Thiol-Monomer und mindestens einem Isocyanat-Monomer umfasst, wobei das mindestens eine Thiol-Monomer 2,5-Dimercaptomethyl-1,4-dithian; 2,3-Dimercapto-1-propanol; 2-Mercaptoethylsulfid; 1,2,3-Trimercaptopropan; Ethylenglykol-bis(thioglycolat); Ethylenglykol-bis(3-mercaptopropionat); Pentaerythritol-tetra(3-mercaptopropionat); Trimethylolpropan-tris(3-mercaptopropionat); Pentaerythritol-tetra(2-mercaptoacetat); Trimethylolpropan-tris(2-mercaptoacetat); 1,6-Hexandithiol; 1,2-Benzoldithiol; 1,3-Benzoldithiol; Isophoron-diurethanthiol; oder eine Kombination der vorgenannten umfasst, wobei das mindestens eine Isocyanat-Monomer Isophoron-diisocyanat; Methylen-dicyclohexyl-diisocyanat; 2,4-Diisocyanattoluol; 4,4'-Methylen-bis-(cyclohexylisocyanat); Hexamethylendiisocyanat; Biuret aus Hexamethylendiisocyanat; Hexamethylendiisocyanat-Isocyanurat-Trimer; Hexamethylendiisocyanat-Uretdion; Poly(hexamethylendiisocyanat); Isophoron-diisocyanat-Trimer; 1,3-Cyclohexan-bis(methylisocyanat); 2,2,4-Trimethylhexamethylendiisocyanat; oder eine Kombination der vorgenannten umfasst, und wobei das mindestens eine (Meth)acrylat-Monomer Methylacrylat; Ethylacrylat; Methylmethacrylat; Acrylsäureanhydrid; Acrylamid; Methacrylamid; Acrylsäure; Methacrylsäure; Methacrylsäureanhydrid; Ethylenglykol-di(meth)acrylat; Tetraethylenglykol-di(meth)acrylat; Poly(ethylenglykol)dimethacrylat; ein Kondensationsprodukt von Bisphenol A und Glycidylmethacrylat; 2,2'-bis[4-(3-methacryloxy-2-hydroxypropoxy)-phenyl]propan, Hexandiol-di(meth)acrylat; Tripropylenglykol-di(meth)acrylat; Butandiol-di(meth)acrylat; Neopentylglykol-di(meth)acrylat; Diethylenglykol-di(meth)acrylat; Triethylenglykol-di(meth)acrylat; Dipropylenglykol-di(meth)acrylat; Allyl(meth)acrylat; Trimethylolpropan-triacrylat; Tricyclodecan-dimethanol-diacrylat; oder eine Kombination der vorgenannten umfasst.

2. Biomaterialharz nach Anspruch 1, wobei das Thiol-Monomer Pentaerythritol-tetra(3-mercaptopropionat), das Isocyanat-Monomer Hexamethylendiisocyanat und das (Meth)acrylat-Monomer Trimethylolpropan-triacrylat ist.

3. Biomaterialharz nach Anspruch 1, wobei das Thiol-Monomer und das Isocyanat-Monomer in einem Verhältnis von Thiol zu Isocyanat gleich oder größer drei kombiniert werden.

4. Biomaterialharz nach Anspruch 1, wobei das Thiourethan-Oligomer weiterhin einen Katalysator umfasst, wobei der Katalysator vorzugsweise Dibutylzinnlaurat; N,N,N',N'-Tetrakis(2-hydroxypropyl)ethylendiamin; 1-[N,N-bis(2-hydroxyethyl)amino]-2-propanol, 4-[N,N-bis(2-hydroxyethyl)amino]benzaldehyd; Triethanolamin; Triisopropanolamin; oder eine Kombination der vorgenannten umfasst.

5. Biomaterialharz nach Anspruch 1, weiterhin umfassend einen Photokatalysator wie 1,2-Dicyclohexyl-4,4,5,5-tetramethylbiguanidium-n-butyltriphenylborat; Benzophenon; oder eine Kombination der vorgenannten.

6. Biomaterialharz nach Anspruch 1, weiterhin umfassend einen Photosensibilisator und/oder ein Lösungsmittel, wobei der Photosensibilisator vorzugsweise 2-Isopropylthioxanthon umfasst und wobei das Lösungsmittel vorzugsweise Aceton oder Dimethylsulfoxid ist.

7. Verfahren zur Herstellung eines Biomaterialharzes nach Anspruch 1, umfassend:
Oligomerisieren eines Gemischs, das mindestens ein Thiol-Monomer und mindestens ein Isocyanat-Monomer zur Bildung eines Thiourethan-Oligomers umfasst; und Kombinieren des Thiourethan-Oligomers mit mindestens einem (Meth)acrylat-Monomer und mindestens einem Photoinitiator und optional mindestens einem Photosensibilisator zur Bildung des Biomaterialharzes, wobei optional das Oligomerisieren in Anwesenheit eines Katalysators erfolgt.

8. Verfahren nach Anspruch 7, wobei das Gemisch ein Verhältnis von Thiol-Einheiten zu Isocyanat-Einheiten von drei oder mehr aufweist.

9. Verfahren zur Herstellung eines Biomaterialnetzwerks, umfassend:
Bereitstellen eines Biomaterialharzes nach Anspruch 1;
Platzieren des Biomaterialharzes in einen Harzaufnahme- oder Abgabebereich eines 3D-Druckers;
Bestrahlen des Biomaterialharzes mit UV- oder sichtbarer Strahlung zur Erzeugung eines polymerisierten Anteilsund eines unpolymerisierten Anteils;
Entfernen des unpolymerisierten Anteilsvom polymerisierten Anteil zur Isolierung des Biomaterialnetzwerks;
und optionales Erhitzen des Biomaterialnetzwerks.

10. Verfahren nach Anspruch 9, weiterhin umfassend ein Bestrahlen des Biomaterialharzes des Biomaterialnetzwerks mit UV- oder sichtbarem Licht durch eine Maske.

11. Verfahren nach Anspruch 10, weiterhin umfassend: Variieren der Dosis des UV- oder sichtbaren Strahlungsdosis relativ zum Biomaterialharz und/oder Biomaterialnetzwerk oder der Position der Maske relativ zum Biomaterialharz und/oder Biomaterialnetzwerk, wobei das Biomaterialnetzwerk einen Gradienten der Vernetzungsdichte aufweist.

12. Biomaterialvorrichtung zur Reparatur oder zum Ersatz von Sehnen, Bändern, Knochen oder Zähnen, wobei die Biomaterialvorrichtung vorzugsweise ein Sehnenersatz ist, umfassend ein Biomaterialnetzwerk hergestellt nach dem Verfahren gemäß Anspruch 9.

13. Biomaterial nach Anspruch 12, wobei das Biomaterialnetzwerk eine Zugfestigkeit von etwa 3 MPa bis etwa 28 MPa und ein Zugmodul von etwa 21 MPa bis etwa 844 MPa aufweist.

## Revendications

1. Résine de biomatériau pour réparation musculo-squelettique, osseuse et/ou dentaire, comprenant un oligomère de thiouréthane, au moins un monomère de (méth)acrylate et au moins un photo-initiateur, dans laquelle l'oligomère de thiouréthane comprend un produit d'addition d'au moins un monomère de thiol, et au moins un monomère d'isocyanate, dans laquelle le monomère de thiol, au moins au nombre de un, comprend le 2,5-dimercaptométhyl-1,4-dithiane ; le 2,3-dimercapto-1-propanol ; le 2-mercapto-éthylsulfure ; le 1,2,3-trimercaptopropane ; le bis(thioglycolate) d'éthylèneglycol ; le *bis*(3-mercaptopropionate) d'éthylèneglycol ; le tétra(3-mercaptopropionate) de pentaérythritol ; le *tris*(3-mercaptopropionate) de triméthylolpropane ; le tétra(2-mercaptoacétate) de pentaérythritol ; le *tris*(2-mercaptoacétate) de triméthylolpropane ; le 1,6-hexanedithiol ; le 1,2-benzènedithiol ; le 1,3-benzènedithiol ; le diuréthane-thiol d'isophorone ; ou toute combinaison de ceux-ci, dans laquelle le monomère d'isocyanate, au moins au nombre de un, comprend le diisocyanate d'isophorone ; le dicyclohexyldiisocyanate de méthylène ; le 2,4-diisocyanatotoluène ; le bis-(cyclohexylisocyanate) de 4,4'-méthylène ; le diisocyanate d'hexaméthylène ; le biuret de diisocyanate d'hexaméthylène ; le trimère d'hexaméthylène-isocyanurate-diisocyanate ; le diisocyanate-uretdione d'hexaméthylène ; le poly(diisocyanate d'hexaméthylène) ; le trimère de diisocyanate d'isophorone ; le bis(méthylisocyanate) de 1,3-cyclohexane ; le diisocyanate de 2,2,4,-triméthylhexaméthylène ; ou toute combinaison de ceux-ci, et dans laquelle le monomère de (méth)acrylate, au moins au nombre de un, comprend l'acrylate de méthyle ; l'acrylate d'éthyle ; le méthacrylate de méthyle ; l'anhydride acrylique ; l'acrylamide ; le méthacrylamide ; l'acide acrylique ; l'acide méthacrylique ; l'anhydride méthacrylique ; le di(méth)acrylate d'éthylèneglycol ; le di(méth)acrylate de tétraéthylèneglycol ; le diméthacrylate de poly(éthylèneglycol) ; un produit de condensation de bisphénol A et de méthacrylate de glycidyle ; le 2,2'-bis[4-(3-méthacryloxy-2-hydroxypropoxy)-phényl]propane ; di(méth)acrylate d'hexanediol ; le di(méth)acrylate de tripropylèneglycol ; le di(méth)acrylate de butanediol ; le di(méth)acrylate de néopentylglycol ; le di(méth)acrylate de diéthylèneglycol ; le di(méth)acrylate de triéthylèneglycol ; le di(méth)acrylate de dipropylèneglycol ; le triméthylolpropane-triacrylate d'allyl(méth)acrylate ; le diacrylate de tricyclodécane-diméthanol ; ou toute combinaison de ceux-ci.

2. Résine de biomatériau selon la revendication 1, dans laquelle le monomère de thiol est le tétra(3-mercaptopropionate) de pentaérythritol, le monomère d'isocyanate est le diisocyanate d'hexaméthylène, et le monomère de (méth)acrylate est le triacrylate de triméthylolpropane.

3. Résine de biomatériau selon la revendication 1, dans laquelle le monomère de thiol et le monomère d'isocyanate sont combinés selon un rapport de thiol à isocyanate supérieur ou égal à trois.

4. Résine de biomatériau selon la revendication 1, dans laquelle l'oligomère de thiouréthane comprend en outre un catalyseur, de préférence dans laquelle le catalyseur comprend du laurate de dibutyl-étain ; de la *N,N,N',N'-tétrakis*(2-hydroxypropyl)éthylènediamine ; du 1-[*N,N*-*bis*(2-hydroxyéthyl)amino]-2-propanol ; du 4-[*N,N-bis*(2-hydroxyéthyl)amino]benzaldéhyde ; de la triéthanolamine ; de la tri-isopropanolamine ; ou toute combinaison de ceux-ci.

5. Résine de biomatériau selon la revendication 1, comprenant en outre un photocatalyseur tel que le n-butyltriphénylborate de 1,2-dicyclohexyl-4,4,5,5-tétraméthylbiguanidium ; la benzophénone ; ou toute combinaison de ceux-ci.

6. Résine de biomatériau selon la revendication 1, comprenant en outre un photosensibilisateur et/ou un solvant, de préférence dans laquelle le photosensibilisateur comprend la 2-isopropylthioxanthone, et de préférence dans laquelle le solvant est l'acétone ou le diméthylsulfoxyde.

7. Procédé de fabrication d'une résine de biomatériau selon la revendication 1, comprenant les étapes consistant à :
oligomériser un mélange comprenant au moins un monomère de thiol et au moins un monomère d'isocyanate pour former un oligomère de thiouréthane ; et
combiner l'oligomère de thiouréthane avec au moins un monomère de (méth)acrylate et au moins un photo-initiateur, et facultativement au moins un photosensibilisateur pour former la résine de biomatériau, facultativement dans lequel l'oligomérisation se fait en présence d'un catalyseur.

8. Procédé selon la revendication 7, dans lequel le mélange comprend un rapport des unités de thiol aux unités d'isocyanate supérieur ou égal à trois.

9. Procédé de formation d'un réseau de biomatériau, comprenant les étapes consistant à :
fournir une résine de biomatériau selon la revendication 1 ;
placer la résine de biomatériau dans un étage de réception ou de livraison de résine d'une imprimante 3D ;
irradier la résine de biomatériau avec un rayonnement UV ou visible pour générer une partie polymérisée et une partie non polymérisée ;
retirer la partie non polymérisée de la partie polymérisée pour isoler le réseau de biomatériau, et
facultativement chauffer le réseau de biomatériau.

10. Procédé selon la revendication 9, comprenant en outre l'irradiation de la résine de biomatériau avec une lumière UV ou visible du réseau de biomatériau à travers un masque.

11. Procédé selon la revendication 10, comprenant en outre : une modification du dosage de rayonnement UV ou de lumière visible par rapport à la résine de biomatériau et/ou au réseau de biomatériau : ou de la position du masque par rapport à la résine de biomatériau et/ou au réseau de biomatériau, le réseau de biomatériau présentant un gradient de densité de réticulation.

12. Dispositif de biomatériau pour la réparation ou le remplacement de tendons, de ligaments, d'os ou de dents, de préférence dans lequel le dispositif de biomatériau est un substitut de tendon, comprenant un réseau de biomatériau formé par le procédé selon la revendication 9.

13. Dispositif de biomatériau selon la revendication 12, dans lequel le réseau de biomatériau présente une résistance à la traction d'environ 3 MPa à environ 28 MPa et un module de traction d'environ 21 MPa à environ 844 MPa.
